(19) Europäisches Patentamt
European Patent Office
Office européen des brevets

(11) Publication number: 0 114 528
A2

# (12) EUROPEAN PATENT APPLICATION

(21) Application number: 83307954.4

(22) Date of filing: 23.12.83

(51) Int. Cl.$^3$: B 01 D 15/08

(30) Priority: 27.12.82 US 453757

(43) Date of publication of application:
01.08.84 Bulletin 84/31

(84) Designated Contracting States:
DE FR GB IT

(71) Applicant: THE TRUSTEES OF BOSTON UNIVERSITY
881 Commonwealth Avenue
Boston, MA 02215(US)

(72) Inventor: Osband, Michael E.
25 Beals Street
Brookline Massachusetts 02146(US)

(72) Inventor: Cohen, Ellen B.
32 Dogwood Lane
Needham Massachusetts 02192(US)

(72) Inventor: Hamilton, Dylan L.
1145 Chapel Street, Apt. 201
New Haven Conecticut 06510(US)

(74) Representative: Holdcroft, James Gerald, Dr. et al,
Graham Watt & Co. Riverhead
Sevenoaks Kent TN13 2BN(GB)

## (54) Cell separation on plates using non-antibody ligands.

(57) Plates, rather than particulate material, are utilized to separate cells into various categories depending upon whether the cells contain receptors that bind to non-antibody ligands, such as histamine. This method comprises coating a plate surface with ligand complexes, which comprise ligands bound to carrier molecules. The coated plates are contacted with cells, and the unbound cells are removed by force which does not remove all bound cells.

EP 0 114 528 A2

# CELL SEPARATION ON PLATES USING NON-ANTIBODY LIGANDS

Certain proteins have the ability to bind non-covalently, and with high specificity, to other molecules which are generally called ligands. The term "ligand" is used inconsistently and can have numerous different meanings. As used herein, the term "ligand" is used to mean a molecule or substance, other than an antibody, which has the following characteristics:

1. A ligand must be capable of binding to some proteinous molecule, such as an antibody, and enzyme, or a receptor which normally exists on the surface of a cell. Such binding may be non-covalent binding.

2. Said binding must have a relatively high degree of specificity; in other words, a ligand has a much higher affinity for its receptor than for other types of receptors. For example, an insulin molecule will bind to an insulin receptor, but not in any substantial degree to a histamine receptor.

One type of ligand binding involves receptors on the surfaces of cells. Mammalian cells have a variety of receptors on their surfaces, such as insulin receptors and histamine receptors. Many such receptors, such as histamine receptors, are proteinous molecules. Other types of receptors comprise e.g. carbohydrates, lectins, glycoproteins.

When a histamine molecule contacts a cells, it does not become bound to the cell unless it contacts a histamine receptor. If this occurs, then the histamine

acts as a ligand which is bound by the histamine receptor. This binding reaction activates or promotes one or more biochemical processes which occur inside the cell. After the process inside the cell commences, the ligand may become detached from the receptor, allowing the receptor to become subsequently bound to another ligand molecule.

As used herein, the term "activator" is used to describe a ligand which binds to a receptor on the surface of a cell. This term is not limited, however, to ligands which activate biochemical processes inside a cell; the term activator includes antagonists and agonists, as those terms are described below. In addition, the term activator includes ligands which terminate or suppress cellular processes by binding to cell receptors.

<u>Histamine</u>

Histamine is a biogenic amine with the following structural configuration:

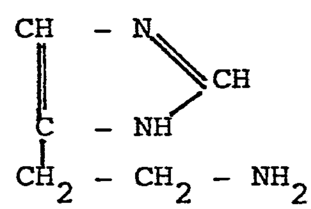

Histamine is secreted by various types of cells, and it may become attached to receptors on the membranes of other cells. The attachment of histamines to histamine receptors can cause or aggravate a wide variety of physiological reactions, including vasodilation, bronchoconstriction, secretion of gastric acids, and urticaria [1]. The term "histamine" as used herein includes substituted forms of histamine, such as methyl histamine.

The effects of histamine may be mimicked by compounds that are referred to as agonists [2]. Histamine agonists include 2(2-pyridyl)ethylamine, dimaprit, and various substituted histamines, such as 4-methylhistamine. Histamine agonists mimic the effect of histamine by attaching to histamine receptors.

The effects of histamine may be blocked by compounds that are referred to as antagonists [3]. Histamine antagonists include diphenhydramine and cimetidine, which block various effects of histamine by attaching to histamine receptors without inducing the physiological effects caused by histamine. However, at high dosage levels, certain antagonists such as impromidine may cause agonistic effects on certain types of cells.

Histamine receptors are believed to be proteinous molecules with molecular weights in the range of 40,000 to 50,000 daltons. They are divided into at least two categories, designated as H1 receptors and H2 receptors [4]. Histamine will bind to either type of receptor. Certain antagonists such as diphenhydramine will bind to and consequently block H1 receptors to a much greater extent than they will block H2 receptors. Other antagonists, including cimetidine, will bind to H2

receptors to a much greater extent than they will bind to H1 receptors. It is believed that binding of histamine to H2 receptors plays an important role in the activation of gastric secretion. Therefore, H2 antagonists such as cimetidine are commonly used to treat ulcers by reducing gastric secretion [5]. It is believed that binding of histamine to H1 receptors plays an important role in skin hives and bronchioconstriction [6]. It is suspected that other categories of histamine receptors exist, which are not blocked by H1 blockers or by H2 blockers. For convenience, such receptors are referred to herein as H3 receptors.

When a histamine, histamine agonist, or histamine antagonist molecule binds to a histamine receptor, the reaction is referred to herein as specific binding. By comparison, if histamine (which is a somewhat reactive molecule) clings or binds to some other part of a cell, a test tube, a filter, or any other surface, such a reaction is referred to herein as non-specific binding.

Histamine blockers, as used herein, is a generic term comprising histamine agonists, histamine antagonists, and other molecules which are capable of specific binding with a histamine receptor, thereby preventing a histamine molecule from specifically binding to the blocked receptor. A histamine blocker which binds to H1 receptors with greater affinity than it binds to H2 receptors is referred to as an "H1 blocker;" for example, diphenhydramine (which is commercially available under the trademark Benadryl (Parke-Davis, Morris Plains, NJ)) is an H1 blocker. Alternately, an H2 blocker such as cimetidine (which is commercailly available under the trademark Tagamet (Smith Kline and French Co., Philadelphia, PA)) binds preferentially to H2 receptors.

Certain diseases are characterized by, and may be caused or aggravated by, imbalances in H1 and H2 receptor density on lymphocytes and other types of human cells. Such diseases include atopic disease [7], neoplastic disease [8], histiocytosis-X [9], autoimmune disease [10], and other diseases [11]. In order to diagnose, study, and treat such diseases it is useful to determine the existence, concentration, and biochemical affinity of H1 and H2 receptors on various types of human cells.

Although it has been recognized since 1910 that histamine causes a variety of physiological reactions, research on histamine receptors has proceeded slowly. The majority of histamine research involved detection of physiological responses, such as muscle contraction or vasodilation, which occur when tissue is contacted with histamine [12]. Some research involved the biochemical production of certain molecules, such as cyclic adenosine monophosphate (cAMP) by cells contacted with histamine [13]. In 1966, it was recognized that mepyramine, when incubated with tissue, blocked certain types of physiological responses but not other types of responses when the tissue was subsequently contacted with histamine. Based upon that result, scientists speculated that at least two types of cell membrane receptors existed, designated as H1 and non-H1 receptors [14]. In 1972, it was discovered that burimamide blocked several responses that differed from the responses blocked by mepyramine; this led to the designation of H2 receptors [15]. Most of the research between 1972 and 1977 focused upon physiological responses or cAMP stimulation [16].

Starting in 1977, research results began to appear which described histamine receptor analyses that utilized biochemical binding reactions [17]. In 1979, Osband et al reported the separation and partial characterization of H1 and H2 receptors based upon the ability of H1 receptors to bind to DEAE-cellulose and the ability of H2 receptors to bind to phosphocellulose [18]. In 1980, Osband et al reported a method, using fluorescent histamine complexes and a flow cytometer, for determining (1) whether a cell contains a substantial number of H1 and/or H2 receptors, and (2) the percentage of cells within a population that contain a substantial number of H1 and/or H2 receptors [19]. In 1981, Osband et al reported a method, using tritiated histamine (which is radioactive), to indicate the number of H1 and/or H2 receptors on a receptor-bearing cell [20].

Ligand Binding

Ligand binding is the basis for a variety of purification techniques, including affinity chromatography. In this technique, ligand molecules are affixed to the surfaces of solid particles which are packed within a column. A mixture of proteins is passed through the porous material within a column. The proteins which have specific affinity for the bound ligands remain within the column; the other proteins pass through the column. For example, histamine molecules can be affixed to agarose or sepharose beads. The coated beads may be packed in a column, and an aqueous solution which contains histamine receptors may be passed through the column. Alternately, the coated beads may be mixed in solution and incubated with the cells. Histamine receptors in the solution

become bound to the histamine-coated beads, while the other receptors and other molecules do not become attached. By washing and/or filtration methods, the unbound (non-histamine) receptors can be separated from the beads and the bound receptors.

The bead-utilizing methods have been used to concentrate, separate, or otherwise study various receptors which were previously detached from cell membranes or which were mixed in solutions that contained cell membrane fragments [21]. In addition, bead-utilizing methods have been used to concentrate, separate, or otherwise study viable cells which contain histamine receptors on their membranes [22]. However, affinity chromatography and other ligand-binding methods which utilize beads or other small particles suffer from several drawbacks in comparison with plates.

As used herein, the terms "plate" or "plate surface" refer to any solid surface which is not comprised of particulate material. Plate surfaces may be planar or curved, and may be smooth or irregular in surface texture. A plate surface must be of a size which can be seen (for example, 1 cm or more in diameter) and is capable of being handled without the use of special equipment such as microscopes. A plurality of plate surfaces may exist on or within a single plate or other device. For example, in a microtiter plate, each microtiter well may be regarded as a plate surface.

In general, plates (such as culture dishes and microtiter plates) tend to be easier to handle, sterilize, and control than beads or packed columns. Plate binding analyses can usually be performed more quickly than analyses using beads. It is more difficult to remove bound cells (which are usually the cells of greatest interest) from beads than from a

plate. For these and other reasons, there are very significant advantages to utilizing plates rather than beads to separate cells.

Efforts have been made to separate cells on flat plates by binding antibodies to the plates [23]. The antibodies are created or selected to bind to antigens on the surfaces of the cells. A suspension of cells is contacted with the antibody-coated plate, and only cells with the relevant antigens become bound to the antibodies on the plate.

However, there are important differences between antibodies and receptor activators. Antibodies are large proteinous molecules with molecular weights in the tens or hundreds of thousands of daltons; by contrast, histamine is a small molecule with a molecular weight of only 111 daltons. Therefore, a small ligand such as histamine may not be able to extend sufficiently far away from the solid surface to allow it to contact cells in solution. This problem is sometimes mitigated by the use of "spacer" molecules between the beads and the ligands [24].

Another difference between antibody binding and ligand binding relates to the fact that antibodies tend to bind very tightly to antigens; antigen-antibody complexes normally do not separate until the constituents are biodegraded <u>in vivo</u>, or are separated by chemical or physical means <u>in vitro</u>. By contrast, a receptor activator may be bound to a receptor for only a brief period, such as a few seconds or less. Many activators dissociate from their receptors fairly quickly, before a cell separation analysis can be completed. This problem is believed to be particularly severe

with respect to small activators, such as histamine and acetylcholine, and less severe with respect to large, proteinous receptors such as insulin.

For these and other reasons, the cell-binding plate techniques that utilize antibodies cannot be converted easily into similar techniques using histamine or other small activators bound to plates.

It is an object of the present invention to provide a method of utilizing plates to separate cells into various categories depending upon whether the cells contain receptors that bind to small molecular ligands, and to assay kits which untilize this method. We have found that advantageously such a method comprises the steps of:

(a) binding a suitable number of ligands, such as histamine or histamine blockers, to a relatively large molecule, referred to herein as a "carrier", to form a ligand complex;

(b) affixing a suitable number of ligand complexes to a plate surface;

(c) contacting the coated plate with a suspension of cells under conditions that allow cells with ligand-specific receptors to become bound to the ligand complexes coated on the plate; and

(d) removing unbound cells by force which is sufficiently strong to remove unbound cells without removing substantially all of the bound cells.

If desired, the bound cells can be subsequently removed

by somewhat stronger force for further analysis. Plates which are coated with ligand complexes as described above may be included in an assay kit.

The invention provides a method of separating cells, characterised in that it comprises the steps of;

(a) contacting a plate surface that is coated with ligand complexes, each of said ligand complexes comprising one or more ligands bound to one or more carrier molecules, with a suspension of cells, at least some of which contain receptors that bind specifically to said ligands, under conditions which allow said ligands to bind to said receptors; and

(b) removing, from said plate surface, cells which are not bound to said ligand complexes by utilizing force which is not sufficiently strong to remove all cells which are bound to said ligand complexes.

The invention also provides a cell separation device comprising a plate surface coated with ligand complexes as defined in the last preceding paragraph.

The invention also provides an assay kit comprising a cell separation device according to the invention and one or more solutions containing one or more molecular species which are capable of specifically binding to one or more of said ligands.

The ligands have, for example, a molecular weight not greater than about 1000.

To confirm the usefulness of the method, various

cell populations which were classified as receptor positive or negative were analyzed for receptor content using a more elaborate technique, flow cytometry. The results of these experiemnts indicate that,

1. a method embodying this invention is capable of isolating receptor-positive cells into a population of cells that contain 5% or less receptor-negative cells, for all types of receptors analyzed, and

2. the method embodying the invention is capable of concentrating the population of $H1R^+$ or $H2R^+$ cells in a population by a factor of at least 4x.

The method has several important advantages including;

1. It is capable of separating cells using plates rather than packed columns or other devices which utilize small beads or particulates.

2. It is capable of utilizing small molecular ligands which are not strongly bound to their receptors.

3. The receptor-bearing cells are viable after they have been separated from the non-receptor bearing cells. After they have been separated from the solid surface, they remain viable, and they have high numbers of functional, unblocked receptors.

There now follows a description of embodiments

of the invention. This description, which is illustrative of method, and apparatus aspects of the invention, is given by way of example only, and not by way of limitation of the invention.

In one preferred embodiment of this invention, histamine and two histamine blockers are utilized to separate cells into the following categories:

$HR^+$ cells: cells which have histamine receptors
$H1R^+$ cells: cells which have H1 receptors
$H2R^+$ cells: cells which have H2 receptors

These are the categories that are currently of greatest interest to most researchers. It is also possible, using the ligand complexes discussed below, to separate or identify cells in the following categories:

$HR^-$ cells: cells which do not have histamine receptors
$H1R^-$ cells: cells which have histamine receptors, but not H1 receptors
$H2R^-$ cells: cells which have histamine receptors, but not H2 receptors
$H1R2R^-$ cells; cells which have histamine receptors, but not H1 or H2 receptors.

Ligand complexes were created by bonding histamine, diphenhydramine (an H1 blocker), or cimetidine (an H2 blocker) to a carrier molecule, human albumin (HA). Approximately 50 to 100 histamine or blocker molecules were added to each carrier molecule. As used herein, the terms "carrier" or "carrier molecule" refer to a molecule with a relatively large molecular weight, such as greater than about 10,000 daltons, to which numerous ligand molecules may be bound. Carrier

molecules may be proteinous, polypeptide, or polymeric molecules, such as albumin, polylysine, or polymethylmethacrylate.

The ligand complexes were diluted in phosphate-buffered saline (PBS), contacted with plastic (polystyrene) plates, and incubated overnight. Excess fluid was poured off; it can be stored and reused. The plates are washed twice with PBS, and they are ready for use. They may be dried, if desired, frozen if desired, and stored for later use. If desired, they may be contacted with a substance such as gelatin to reduce the amount of non-specific binding.

A suspension of cells is contacted with the plates. The cells may be suspended in various fluids, such as PBS or various culture media such as RPMI 1640. The fluid may be supplemented if desired by various substances, such as one or more blood components, such as fetal bovine serum (FBS). The suitability of any fluid or any fluid supplement may be determined through routine experimentation by those skilled in the art.

The cells are incubated in contact with the coated plate for a suitable period of time at a suitable temperature. In the experiments described below, incubation was performed for 1 hour at 37°C; either parameter may be varied if desired. If a large volume of suspension is utilized, the incubation period may be extended to allow the cells to settle and contact the plate.

The plates are then washed with a suitable fluid, such as PBS, to remove cells. A typical washing series is conducted as follows. PBS at 37°C is gently administered to the plate. If desired, the PBS may contain calcium and magnesium, which are believed to promote the binding of receptor-positive cells to ligands. The plate is gently swirled. The PBS is poured off; the large majority of cells in that washing will not have substantial numbers of receptors specific for the type of ligand on the plate. For example, if the plate is coated with histamine-HA complexes, the cells removed by the first wash will be $HR^-$ cells; if the plate is coated with diphenhydramine complexes, the cells removed by the first wash will be $H1R^-$ cells. These cells may be analyzed further if desired.

Subsequent washings, using warm PBS with Ca and Mg, may be performed if desired. The PBS is swirled on the plate, poured off, and analyzed by a cell counter to determine how many cells it contains. When the cell count drops to less than a predetermined value, such as about 5% of the number of cells contacted with the plate, this series of washings is discontinued. Alternately, a fixed number of washings may be performed. The predetermined value or the number of washings depend upon the subsequent use or analysis intended for the cells; for example, if the purpose of the separation is to obtain relatively pure receptor-positive cells, a large number of washings may be performed.

After the foregoing series of washings is completed, cells which remained bound to the plate during those washings may be removed. This may be accomplished by several means, such as gently scraping the plate with a device having a soft edge, e.g., a rubber spatula. Cold (4°C) PBS which does not contain Ca or Mg may be used. Vigorous agitation, such as vortexing or spraying liquid in a jet stream may be used. The preferred method may depend on the subsequent use or analysis intended for the cells.

The accuracy of the method is related to the fraction or number of non-homogeneous cells in the population that is desired, e.g., the fraction or number of $HR^-$ cells in an $HR^+$ population. To a lesser extent, the accuracy is also related to the number of desired cells that are washed off in the washing that are discarded. With respect to both factors, the accuracy tends to be somewhat less than the accuracy of more complex methods such as column separation, or FACS. However, the advantage in terms of lower cost, faster performance, and greater simplicity and convenience, greatly outweighs the disadvantage of slightly reduced accuracy.

Several parameters are important to the proper performance of the method including:

1. The number of ligand molecules per carrier molecule. This ratio may be controlled by several means, such as varying the ratio of ligand molecules to carrier molecules during the coupling reaction, and varying the incubation period of the coupling reaction. The ideal ligand/carrier ratio for a specific use will depend upon several factors, including

the type of ligand molecule, the type of carrier molecule, and the type of cells being analyzed. In general, if the ligand/carrier ratio is too low, receptor-positive cells tend to be detached from the plate by gentle washing, which is undesirable. If the ligand/carrier ratio is too high, receptor-negative cells tend to cling to the plate, which is also undesirable. The optimal ligand/carrier ratio for any specific use may be determined through routine experimentation. In the experiments described, 50 to 100 histamine ligand molecules were conjugated to each albumin carrier molecules.

2. The number of ligand complexes per unit area of plate surface. If the concentration is too high, receptor-negative cells tend to adhere to the plate, while if the concentration is too low, receptor-positive cells tend to become detached from the plate. This concentration may be controlled by several methods, such as varying the number of ligand complexes that are contacted with the plate, and varying the contact time before the plate is poured or rinsed off. The optimal concentration for any specific use may be determined through routine experimentation.

3. The composition of the plate. Numerous types of plastic and other compounds with various binding characteristics are known to those skilled in the art. In addition, methods for coating plastic and other surfaces with molecules that have various functional groups are known to those skilled in the art. The optimal type of plate and/or coating for use with any specific type of ligand may be determined through routine experimentation.

## EXAMPLES

### Example 1; Preparation and Fixation of Lymphocytes

Human lymphocytes were separated from blood samples collected in preservative-free heparin, by centrifugation using Lymphocyte Separation Medium (Bionetics Lab Products, Kensington, MD). T cells were isolated by rosetting lymphocytes with sheep erythrocytes. Some of the cells were fixed by suspension in phosphate-buffered saline (PBS) containing 1% formaldehyde. Fresh cells were used within six hours of preparation; fixed cells were stored at 4°C until use.

### Example 2: Preparation of Ligand Complexes

Human albumin was dissolved in water (25 g albumin per 100 ml of solution), stablilized by 0.02 M sodium coprylate and 0.02 M sodium acetyl-DL-tryptophonate (Mass. Public Health Laboratories, Boston MA). Varying quantities of histamine dihydrochloride (Sigma Chemical Co., St. Louis MO) and a coupling agent, 1-ethyl-3 (3-dimethylamino-propryl)-carbodiimide hydrochloride (ECDI) (Sigma Chemical Co.) were added to 5 ml of the albumin solution. During this process, the ratio of ECDI to histamine (mg/mg) was maintained at about 10:1.

The conjugation of histamine dihydrochloride to albumin was allowed to proceed for two hours at room temperature. A tritiated histamine tracer (New England Nuclear, Boston MA) was added to certain batches to enable monitoring of the amount of histamine conjugated to albumin. Following the conjugation reaction, each solution was dialyzed twice against a 300-fold volume of water, and once against a 300-fold volume of normal saline, to remove unconjugated histamine and ECDI. All dialyses were performed for 90 minutes at

4°C with a stirrer. The solution was adjusted to a final albumin concentration of 3.3 mg/ml in normal saline before use. If desired for storage, the conjugates may be lyophilized and resuspended at a desired albumin concentration, such as 20 mg/ml.

Control human albumin without histamine or histamine blocker was reacted with ECDI and processed in a manner identical to the HAC complexes.

Example 3: Preparation of Albumin-Cimetidine Complexes

400 µl of 25% human serum albumin (100 mg of albumin) was mixed with 4.6 ml of PBS. The temperature was reduced to 4°C. 200 mg of ECDI is dissolved in the solution, and the solution is incubated at 4°C for 5 to 10 minutes. 250 mg of cimetidine (Smith, Kline and French Laboratories, Philadelphia PA) was dissolved in 750 µl 1N HCl, which was adjusted to pH 7.4 with 1 N NaOH. The cimetidine and albumin solutions were mixed at room temperature and allowed to incubate for two hours. The mixture was dialyzed twice against distilled water at 4°C, and against PBS. The final solution contained 100 mg of albumin in 6.5 ml of solution.

Example 4: Preparation of Albumin-Diphenhydramine Complexes

Human serum albumin (as described in Example 1) was diluted with PBS to provide a solution of 5% albumin. 2 g of ECDI were dissolved in 2 ml of the 5% solution. 1 g of diphenhydramine hydrochloride (Sigma Chemical Co., St. Louis MO) and 2 ml normal saline were added. The solution was mixed and allowed to incubate at room temperature for 2 hours, then dialyzed twice against distilled water at 4°C, then dialyzed against PBS. The final solution contained about 100 mg of albumin in about 5 ml of solution.

0114528

Example 5: Binding of Ligand Complexes to Plates

Ligand complexes were prepared as described in Examples 2, 3 and 4, above. These complexes were contacted with polystyrene Corning 100 mm tissue culture plates (Fisher Scientific Co., Pittsburgh, PA). The plates were incubated overnight. The excess fluid was poured off, and the plates were washed once with PBS.

If desired, the plates may be contacted with a solution containing protein, such as a 0.1% solution of gelatin or a 1% solution of bovine serum albumin, which does not contain ligand molecules. The purpose of this step is to coat any exposed plastic surfaces that were not coated by ligand complexes. This reduces non-specific binding of cells to the plates.

Example 6: Binding of Cells to Plates

Cells prepared as described in Example 1, were suspended in RPMI 1640 culture medium, supplemented with 7% heat-inactivated fetal bovine serum, 2 mM calcium, and 1 mM magnesium. The suspension was adjusted if desired by dilution or filtration to a concentration of about 4 or 5 million cells/ml.

The suspension was contacted with plates which were coated with ligand complexes as described in Example 5, at a contact density of about 30 to 60 million cells per 100 mm diameter plate. The plates were incubated for 1 hour at 37°C.

Several competitive binding reactions were performed as described in the following example reaction. To determine the number of cells with H2 receptors, an excess quantity of an H1 blocker, such as diphenhydramine hydrochloride (unattached to protein such as albumin) was mixed with the cell suspension. The diphenhydramine became bound to most of the H1 receptors on the cells, leaving H2 receptors exposed. Since the H1 receptors were blocked, only cells with non-H1 receptors (predominately H2 receptors) became bound to the plate. Other experiments utilized an excess of H2 blocker, such as cimetidine, to determine the number of cells with H1 receptors.

Alternately, if a ligand complex comprising diphenhydramine is bound to the plate, only cells with H1 receptors will bind to the ligand complexes.

Example 7: Removal and Analysis of Cells from Plates

A PBS washing was used to remove the large majority of unbound cells from the plates, while leaving the large majority of bound cells bound to the plates. A typical washing procedure was conducted as follows: 7 ml of warm (37°C) PBS containing 2mM Ca, 1mM Mg, and 7.5% fetal bovine serum was added to the plate by pipette. The plate was swirled, and the PBS was poured off. The washing was repeated. The number of cells in each PBS wash was counted by a cell counter. When the washing contained less than 5% of the number of cells that had been contacted with the plate, then the washings were terminated. All cells washed off by those washings were regarded as $HR^-$ cells. The cells were analyzed by flow cytometric methods described by M. Osband et al, Blood, Vo. 56, No. 5: 923-925 (1980).

Cells which remained affixed to the plate after the PBS washing was completed were removed from the plate by placing cold (4°C) PBS without Ca or Mg on the plate and scraping the plate gently with a soft rubber spatula. These cells were analyzed by flow cytometric methods cited above.

The results of these experiments indicate that, although there is a great deal of variation between the numbers of histamine receptors on different types of cells, the method is consistently capable of isolating receptor-positive cells into a population of cells that contain 5% or less receptor-negative cells, for all types of receptors analyzed. In addition, the method of this invention is consistently capable of concentrating the population of $H1R^+$ or $H2R^+$ cells in a population by a factor of at least 4x.

## REFERENCES

1. See, e.g., T. O. Yellin, Editor, Histamine Receptors, SP Medical and Scientific Books, New York (1979).

2. See, e.g., M.E. Parsons et al,"3-[4(5)-Imidazolyl] propylguanidine - a Partial Agonist at Histamine H2 Receptors," Agents Actions 5: 464 (1975).

3. See, e.g., J. W. Black et al, "Metiamide - an Orally Active Histamine H2 Receptor Antagonist," Agents Actions 3: 133-137 (1973).

4. See, e.g., M. Osband et al, "Solubilization, Separation, and Partial Characterization of Histamine H1 and H2 Receptors From Calf Thymocyte Membranes", J. Biol. Chem. 254: 9970-9972 (1979).

5. See, e.g., R. R. Schade, "How Physicians Use Cimetidine", N. Eng. J. Med. 304: 1281-1284 (1981).

6. See, e.g., A. S. F. Ash et al, "Receptors Mediating Some Actions of Histamine," Brit. J. Pharmacol. Chemotherap. 27: 427-439 (1966).

7. See, e.g., J. D. Martinez et al, "Non-specific Suppressor Cell Function in Atopic Subjects", J. Am. Clin. Imm. 64: 485 (1979).

8. See, e.g., B. Schechter, "Enhancing Lymphocytes in Spleen of Tumor-bearing Mice", Int. J. Cancer 20: 239 (1977).

9. M. E. Osband, "Histiocytosis-X", N. Eng. J. Med. 304: 146-153 (1981).

10. W. DeCock et al, "Histamine Receptor-bearing T Lymphocytes in Patients with Allergy, Autoimmune Disease, or Recurrent Infection", Clin. Immunol. Immunopath. 11: 1 (1978).

11. See, e.g., A. Gupta, "Markers of Human Lymphocyte Subpopulations in Primary Immunodeficiency and Lymphoproliferative Disorders", Semin. Hemat. 17: 1 (1980)

12. See, e.g., M. A. Beaven, Histamine, Its Role in Physiological and Pathological Processes: 36-51 (S. Carger, Basel, Switzerland, 1978).

13. See, e.g., H. Shimizu et al, "The Effect of Histamines and Other Compounds on the Formation of AMP in Slices from Cerebral Cortex", J. Neurochemistry 17: 441-444 (1970)

14. See Ash et al, supra note 6.

15. J. W. Black et al, "Definition and Antagonism of Histamine H2 Receptors", Nature 236: 385-390 (1972).

16. See Yellin, supra note 1.

17. S. J. Hill et al, "Specific Binding of $^3$H-mepyramine to Histamine H1 Receptors in Intestinal Smooth Muscle", Nature 270: p. 361-363 (1977); W. P. Burkard, "Histamine H2 Receptor Binding with $^3$H-cimetidine in Brain", Euro. J. Pharmacol. 50: 449-450 (1978).

18. Osband et al, supra note 4.

19. M. E. Osband et al, "A Technique for Flow Cytometric Analysis of Lymphocytes Bearing Histamine Receptors," Blood 56 #5: 923-925 (1980).

20. M. E. Osband et al, "Biochemical Analysis of Specific Histamine $H_1$ and $H_2$ Receptors on Lymphocytes," Blood 58 #1: 87-90 (1981).

21. See, e.g., P. Cuatrecasas, Proc. Natl. Acad. Sci. USA 69: 1277 (1972).

22. Y. Weinstein et al, "Specific Leukocyte Receptors for Small Endogenous Hormones...", J. Clinical Investigation 52: 1349-1361 (1973); K. L. Melmon et al, "Separation of Specific Antibody-Forming Mouse Cells by their Adherence to Insolubilized Endogenous Hormones," J. Clinical Investigation 53: 22-30 (1974).

23. See, e.g., L. J. Wysocki et al, "'Panning' for Lymphocytes: A Method for Cell Selection," Proc. Natl. Acad. Sci. USA 75 #6: 2844-2848 (1978).

24. See, e.g., A. L. Lehninger, Biochemistry, 2nd edition, 169, 821 (Worth Publ., San Francisco CA, 1975).

CLAIMS:

1. A method of separating cells, characterised in that it comprises the steps of:

a) contacting a plate surface that is coated with ligand complexes, each of said ligand complexes comprising one or more ligands bound to one or more carrier molecules, with a suspension of cells, at least some of which contain receptors that bind specifically to said ligands, under conditions which allow said ligands to bind to said receptors; and

b) removing, from said plate surface, cells which are not bound to said ligand complexes by utilizing force which is not sufficiently strong to remove all cells which are bound to said ligand complexes.

2. A method according to claim 1, wherein said carrier molecules comprise albumin.

3. A method according to claim 1 or claim 2, wherein said ligands have a molecular weight not greater than about 1,000.

4. A method according to any one of claims 1, 2 and 3, wherein said ligands bind specifically to histamine receptors.

5. A method according to claim 1 or claim 2, wherein said ligands are selected from histamine, diphenhydramine, and cimetidine.

6. A method according to any one of the preceding claims, wherein 50 to 100 ligands are bound to each carrier molecule.

7. A method according to any one of the preceding claims, wherein said plate surface coated with ligand complexes is subsequently contacted with a substance (e.g. gelatin or albumin) that minimizes non-specific binding of cells to the plate surface, prior to contacting said plate surface with cells.

8. A cell separation device comprising a plate surface coated with ligand complexes as defined in any one of claims 1 to 6.

9. A device according to claim 8, also coated with a substance (e.g. gelatin or albumin) that minimizes non-specific binding of cells to the plate surface.

10. An assay kit comprising a cell separation device according to claim 8 or claim 9 and one or more solutions containing one or more molecular species which are capable of specifically binding to one or more of said ligands.